# EUROPEAN PATENT APPLICATION

(11) **EP 2 397 039 A1**
(43) Date of publication of application: **21.12.2011**
(21) Application number: 10380083.5
(22) Date of filing: 21.06.2010
(51) Int. Cl.: A23L 1/30, A61K 36/37

(54) **Compositions for delaying progression of diabetes using Salacia oblonga extract**

(71) Applicant: Abbott Laboratories, Columbus OH 43219 (US)
(72) Inventor: Rueda Cabrera, Ricardo, 18008 Granada (ES); Manzano Martin, Manuel, 18151 Ogijares (Granada) (ES); Lopez Pedrosa, Jose Maria, 18009 Granada (ES)
(74) Representative: Ungria Lopez, Javier

(57) **Abstract**

Disclosed are nutritional compositions and methods for delaying the onset or progression of type 2 diabetes in a subject. The methods include administering to a glucose intolerant or diabetic subject a nutritional composition comprising an effective amount of a *Salacia oblonga* extract for increasing the incretin effect (and/or the glucagon-like peptide-1 and/or gastric inhibitory peptide activity) in the subject. The *Salacia oblonga* extract may also act to reduce the secretion of glucagon by the pancreas thus decreasing the glucose level in the bloodstream of the subject.

## Description

### TECHNICAL FIELD

The present disclosure relates to methods for delaying the progression of diabetes using nutritional compositions including a *Salacia oblonga* extract. The present disclosure further relates to methods for increasing insulin secretion in a glucose-dependent manner and/or decreasing glucagon secretion through enhancing the insulin-potentiating effects of the incretin hormones in individuals afflicted with impaired glucose tolerance or diabetes using nutritional compositions including a *Salacia oblonga* extract.

### BACKGROUND OF THE DISCLOSURE

Diabetes mellitus is a disorder of carbohydrate metabolism resulting from insufficient production of, or reduced sensitivity to, insulin. In persons who have diabetes, the normal ability of body cells to use glucose is inhibited, thereby increasing blood sugar levels. As more glucose accumulates in the blood, excess levels of sugar are excreted in the urine. Corresponding symptoms of diabetes include increased urinary volume and frequency, thirst, hunger, weight loss, and weakness.

There are two variations of diabetes. Type 1 diabetes is insulin dependent diabetes mellitus for which insulin administration is required. In a subject patient with type 1 diabetes, insulin is not secreted by the pancreas and therefore must be taken by injection or inhalation.

Type 2 diabetes may be controlled by dietary restriction, oral anti-hyperglycemic agents, and/or insulin administration. Type 2 diabetes can be attributable to dilatory pancreatic secretion of insulin and reduced sensitivity to the action of insulin on target tissues.

First phase insulin secretion, the typical response to the increase in blood glucose level after consumption of a meal, (which contributes to about 50% of the posprandial hyperglycemic excursion), is reduced or absent in patients with type 2 diabetes, and is among the earliest abnormalities of type 2 diabetes. Degeneration of the first-phase insulin response is a marker of b-cell failure and portends conditions that are like the progression from IGT to T2DM. In healthy persons, the stimulating insulin secretion to an oral glucose load is considerably greater than that to an intravenous glucose infusion at an amount able to induce a comparable profile of blood glucose concentration. This increase on glucose-stimulated insulin secretion by oral glucose is defined as incretin effect and is mediated by two intestinally derived peptides, the glucose-dependent insulinotropic polypeptide (GIP) and the glucagon-like peptide 1 (GLP-1). Additionally, GLP-1 significantly influences the islet function, including first phase insulin secretion. In patients with type 2 diabetes, decreased secretion of GLP-1 leads to an impaired incretin effect, which contributes to the postprandial hyperglycemia and diabetes progression. Although current orally administered antidiabetic products are effective during the course of type 2 diabetes, most patients ultimately require insulin because of progressive β-cell failure.

Complications from diabetes often involve the cardiovascular system, which then accounts for the majority of diabetes-related deaths. Other serious complications include diabetic retinopathy, kidney disease, peripheral neuropathy, and/or frequent infection.

As such, there is a need for nutritional compositions and methods for treating and managing diabetes, as well as impaired glucose tolerance. It would be beneficial if such compositions and treatments could be based on naturally occurring plant species so as to potentially limit the side effects of synthetic medications.

### SUMMARY OF THE DISCLOSURE

One embodiment is directed to a method of delaying the progression of type 2 diabetes in a subject. The method comprises administering to the subject a nutritional composition comprising an effective amount of a *Salacia oblonga* extract.

Another embodiment is directed to a method of increasing the incretin effect in an individual afflicted with impaired glucose tolerance or diabetes. The method comprises administering to the subject a nutritional composition comprising an effective amount of a *Salacia oblonga* extract.

Another embodiment is directed to a method of increasing glucagon-like peptide-1 activity in an individual afflicted with impaired glucose tolerance or diabetes. The method comprises administering to the subject a nutritional composition comprising an effective amount of a *Salacia oblonga* extract.

Another embodiment is directed to a method of suppressing glucagon secretion in a subject. The method comprises administering to the subject a nutritional composition comprising an effective amount of a *Salacia oblonga* extract.

It has now been discovered that *Salacia oblonga* extract may delay the onset or progression of type 2 diabetes in individuals with impaired glucose tolerance or diabetes. It appears that the *Salacia oblonga* extract may restore, increase and/or stimulate the effects of incretin hormones, and specifically GLP-1 and/or GIP, to healthy or near-healthy levels, thus changing the onset or progression of type 2 diabetes and achieving long-term efficacy. By affecting the incretin levels, the nutritional compositions and methods of the present disclosure help maintain the capacity of pancreatic β-cells to synthesize and secrete insulin in response to glucose, may increase β-cell mass, and suppress glucagon secretion, all of which help to delay type 2 diabetes progression.

Accordingly, the nutritional compositions and methods of the present disclosure offer a therapeutic option that may contribute to the maintenance of optimal glycemic control and to preserve β-cell function in subjects that are prediabetic, have impaired glucose tolerance, or have type 2 diabetes. Further, these nutritional compositions may be able to slow the progression of type 2 diabetes. These benefits are advantageously achieved without the complications seen with the previously used oral synthetic pharmacological approaches.

### BRIEF DESCRIPTION OF THE DRAWINGS

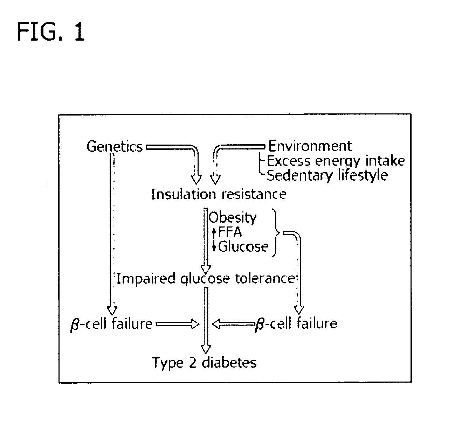

Figure 1 is a flow chart depicting the progression of increasing insulin resistance and β-cell failure to type 2 diabetes.
Figure 2 depicts postprandial glycemic response of obese Zucker rats after oral meal load with maltodextrin with (green) or without (red) the concomitant ingestion of *Salacia oblonga* extract (150mg/kg body weight) as tested in Example 1.
Figure 3 depicts postprandial glycemic response during the time course of an oral meal load with maltodextrin in obese Zucker rats fed with a standard purified diet or the same diet supplemented with *Salacia oblonga* extract as tested in Example 1.
Figures 4 (A) and (B) depict fasting insulin concentrations (A) and postprandial insulin response (B) during the time course of an oral meal load with maltodextrin in obese Zucker rats fed with a standard purified diet or the same diet supplemented with *Salacia oblonga* extract as tested in Example 1.
Figures 5 (A) and (B) depict incremental area under the curves for insulin concentrations, (A) between 0 to 30 min, (B) between 0 and 180 min post-gavage, during the time course of an oral meal load with maltodextrin in obese Zucker rats fed with a standard purified diet or the same diet supplemented with *Salacia oblonga* extract as tested in Example 1.
Figure 6 depicts the change in body weight of genetically obese Zucker rats fed with standard purified diet or the same diet supplemented with *Salacia oblonga* extract as tested in Example 2.
Figure 7 depicts the fasting glucose and insulin concentrations of obese Zucker rats fed with a standard purified diet or the same diet supplemented with *Salacia oblonga* extract as tested in Example 2.
Figure 8 depicts the whole-body insulin sensitivity, measured by the rate of glucose infusion in the steady state, of obese Zucker rats fed with standard purified diet or the same diet supplemented with *Salacia oblonga* extract as tested in Example 2.
Figure 9 depicts the change in body weight of genetically obese Zucker rats fed with a standard purified diet or the same diet supplemented with *Salacia oblonga* extract as tested in Example 3.
Figure 10 depicts the fasting glucose and insulin concentrations of obese Zucker rats fed with a standard purified diet or the same diet supplemented with *Salacia oblonga* extract as tested in Example 3.
Figure 11A depicts glucose infusion rate (GIR) over time during hyperglycemic clamp in obese Zucker rats fed with a standard purified diet or the same diet supplemented with *Salacia oblonga* extract as tested in Example 3.
Figure 11B depicts a bar chart of steady state GIR during hyperglycemic clamp in obese Zucker rats fed with a standard purified diet or the same diet supplemented with *Salacia oblonga* extract as tested in Example 3.
Figure 12A depicts serum insulin concentrations during hyperglycemic clamp in obese Zucker rats fed with a standard purified diet or the same diet supplemented with *Salacia oblonga* extract as tested in Example 3.
Figure 12B depicts early phase serum insulin concentrations during hyperglycemic clamp in obese Zucker rats fed with a standard purified diet or the same diet supplemented with *Salacia oblonga* extract as tested in Example 3.
Figure 12C depicts late phase serum insulin concentrations during hyperglycemic clamp in obese Zucker rats fed with a standard purified diet or the same diet supplemented with *Salacia oblonga* extract as tested in Example 3.
Figure 13 depicts GIR/Insulin during 20 to 120 minutes of the hyperglycemic clamp period in obese Zucker rats fed with a standard purified diet or the same diet supplemented with *Salacia oblonga* extract as tested in Example 3.
Figure 14 depicts the *in vitro* effect of *Salacia oblonga* on insulin secretion from isolated obese Zucker rats (OZR) islets as tested in Example 4.

### DETAILED DESCRIPTION OF THE DISCLOSURE

The methods disclosed herein are directed to methods utilizing nutritional compositions comprising an effective amount of *Salacia oblonga* extract for providing chronic treatment to individuals with impaired glucose tolerance, prediabetic individuals, and individuals with type 2 diabetes. The essential or optional elements or features of the various embodiments are described in detail hereinafter.

The term "nutritional composition" means the referenced material comprises at least one of fat, protein, and carbohydrate and is suitable for oral administration to a human. The nutritional composition may further comprise vitamins, minerals and other ingredients and represent a sole, primary, or supplemental source of nutrition.

The term "incretin" or "incretins" refers to a group of gastrointestinal hormones including glucagon-like peptide-1 (GLP-1) and Gastric inhibitory peptide (GIP) that cause an increase in the amount of insulin released from the β-cells of the islets of Langerhans after eating.

The term "effective amount" refers to the amount of *Salacia oblonga* extract sufficient for stimulating, restoring, or increasing the incretin effect and/or GLP-1 and/or GIP activity or decreasing glucagon secretion in a person with impaired glucose tolerance or diabetes.

All percentages, parts and ratios as used herein are by weight of the total composition, unless otherwise specified. All such weights as they pertain to listed ingredients are based on the active level and, therefore, do not include solvents or byproducts that may be included in commercially available materials, unless otherwise specified.

All numerical ranges as used herein, whether or not expressly preceded by the term "about", are intended and understood to be preceded by that term, unless otherwise specified.

The compositions and methods herein may also be free of any optional or other ingredient or feature described herein provided that the remaining composition still contains the requisite ingredients or features as described herein. In this context, the term "free" means the selected composition or method contains or is directed to less than a functional amount of the ingredient or feature, typically less than 0.1 % by weight, and also including zero percent by weight, of such ingredient or feature.

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 2 to 8, from 3 to 7, from 5 to 6, from 1 to 9, from 3.6 to 4.6, from 3.5 to 9.9, and so forth.

Any reference to singular characteristics or limitations of the present disclosure shall include the corresponding plural characteristic or limitation, and vice versa, unless otherwise specified or clearly implied to the contrary by the context in which the reference is made.

Any combination of method or process steps as used herein may be performed in any order, unless otherwise specifically or clearly implied to the contrary by the context in which the referenced combination is made.

The nutritional compositions and methods may comprise, consist of, or consist essentially of the elements and features of the disclosure described herein, as well as any additional or optional ingredients, components, or features described herein or otherwise useful in a nutritional application.

### Product Form

The nutritional compositions utilized in the methods of the present disclosure may be formulated in any known or otherwise suitable product form for oral administration. Oral product forms are preferred and include any solid, liquid, or powder formulation suitable for use herein, provided that such a formulation allows for safe and effective oral delivery of the essential and other selected ingredients from the selected product form.

Non-limiting examples of solid nutritional product forms suitable for use herein include snack and meal replacement products, including those formulated as bars, sticks, cookies or breads or cakes or other baked goods, frozen liquids, candy, breakfast cereals, powders or granulated solids or other particulates, snack chips or bites, frozen or retorted entrees and so forth.

Non-limiting examples of liquid product forms suitable for use herein include snack and meal replacement products, hot or cold beverages, carbonated or non carbonated beverages, juices or other acidified beverages, milk or soy-based beverages, shakes, coffees, teas, enteral feeding compositions, and so forth. These liquid compositions are most typically formulated as suspensions or emulsions, but can also be formulated in any other suitable form such as solutions, liquid gels, and so forth.

Other non-limiting examples of suitable oral product forms include semi-solid or semi-liquid compositions (e.g., puddings, gels), as well as more conventional product forms such as capsules, tablets, caplets, pills, and so forth. The quantity of the composition for providing an effective amount of the defined *Salacia oblonga* extract to the targeted user may be contained in one or a plurality of individual dosage forms, e.g., in one tablet or a plurality of tablets.

For product forms such as lozenges, tablets (e.g. chewable, coated, etc.) pastes, or gels, the *Salacia oblonga* extract may be formulated at concentrations most typically ranging from about 0.1 % to about 50%, including from about 0.1 % to about 33%, and also including from about 0.1 % to about 25%, by weight of the product form, all in combination with excipients or other ingredients such as carbohydrates, acidulants, flavors, and colors. The carbohydrate in these product forms preferably contains a non-reducing sugar, concentrations of which may range from about 5 to 100% by weight of the carbohydrate. Non-limiting examples of acidulants in these embodiments include citric acid, malic acid, tartaric acid, lactic acid, or combinations thereof, to enhance salivation and to provide taste masking for bitter or brothy notes.

### Salacia oblonga Extract

The nutritional compositions and methods of the present disclosure utilize a *Salacia oblonga* extract, which contains the α-glucosidase inhibitors salacinol, kotalanol and mangiferin 9. In one embodiment of the present disclosure an amount of *Salacia oblonga* extract is included in a nutritional composition so as to stimulate, restore and/or increase the incretin effect of a person with glucose intolerance, a prediabetic, or type 2 diabetic subject upon administration. In some embodiments, the administration of the *Salacia oblonga* extract to a person with glucose intolerance, a prediabetic or type 2 diabetic subject may restore the incretin effect to levels of that of a healthy person. Additionally, the nutritional compositions including the *Salacia oblonga* extract may be used to reduce the secretion of glucagon from α-cells to help control glucose concentration in the bloodstream.

Suitable *Salacia oblonga* extracts for use in the nutritional compositions and methods of the present disclosure include both powdered and liquid forms of *Salacia oblonga* extracts. One specific example of a suitable *Salacia oblonga* extract is *Salacia oblonga* Extract A or *Salacia oblonga* Extract D (both powdered forms), commercially available from Tanabe Seiyaku Company Limited (Osaka Japan).

In one embodiment, the amount *of Salacia oblonga* extract included in the nutritional composition is an amount effective to reduce insulin concentrations in fasting states of the subject by at least about 10%, including at least about 20%, including at least about 30%, including at least about 50%, and also including reducing insulin concentrations by from about 20% to about 40% when administered daily for a period of at least eight weeks.

Typically, the amount of *Salacia oblonga* extract included in the nutritional compositions of the present disclosure is at least about 0.001% (by weight nutritional composition). In some embodiments the amount of *Salacia oblonga* extract is from about 0.001% (by weight nutritional composition) to about 1.0% (by weight nutritional composition), including from about 0.001% (by weight nutritional composition) to about 0.25% (by weight nutritional composition), and also including from about 0.005% (by weight nutritional composition) to about 0.2%.(by weight nutritional composition).

In some embodiments, the *Salacia oblonga* extract is included in the nutritional compositions as described herein in sufficient amounts to as to provide the user with at least about 40 mg/kg body weight per day *Salacia oblonga* extract, or 75 mg/kg body weight per day *Salacia oblonga* extract, or 100 mg/kg body weight per day *Salacia oblonga* extract, or even 150 mg/kg body weight per day *Salacia oblonga* extract, or even 175 mg/kg body weight per day *Salacia oblonga* extract, or even 200 mg/kg body weight per day *Salacia oblonga* extract. It will be recognized by one skilled in the art based on the disclosure herein that the exact amount of *Salacia oblonga* required by a specific individual to receive the benefits described herein will depend upon a number of factors including, for example, the specific medical condition of the individual.

It has been found that chronic administration of the nutritional compositions including *Salacia oblonga* extract stimulates the initial phase of insulin release; that is, this extract stimulates insulin secretion in a rapid response to the increase in blood glucose levels after consumption of a meal. As used herein, "chronic administration" refers to frequent administration (i.e. daily) for a sustained period of time. The sustained period of time may be, for example, at least one week, at least two weeks, at least three weeks, at least four weeks, at least five weeks, at least six weeks, at least seven weeks, at least two months, at least three months, at least six months, at least one year, or even longer.

### Macronutrients

The compositions of the present disclosure may further comprise one or more macronutrients including a fat source, a carbohydrate source, and a protein source, all in addition to the *Salacia oblonga* extract as defined herein.

The optional macronutrients in combination with the other essential or added ingredients may provide up to about 1000 kcal of energy per serving or dose, including from about 25 kcal to about 900 kcal, also including from about 75 kcal to about 700 kcal, also including from about 100 kcal to about 500 kcal, also including from about 150 kcal to about 400 kcal, and also including from about 200 kcal to about 300 kcal, per serving or dose, preferably as a single, undivided serving or dose.

Many different sources and types of proteins, lipids, and carbohydrates are known and can be used in the various products described herein, provided that the selected nutrients are safe and effective for oral administration and are compatible with the essential and other added ingredients.

Carbohydrates suitable for use in the compositions of the present disclosure may be simple, complex, or variations or combinations thereof. Non-limiting examples of suitable carbohydrates include hydrolyzed or modified starch or cornstarch, maltodextrin, glucose polymers, sucrose, corn syrup, corn syrup solids, rice-derived carbohydrate, glucose, fructose, lactose, high fructose corn syrup, indigestible oligosaccharides (e.g., fructooligosaccharides), soluble or insoluble fiber, honey, sugar alcohols (e.g., maltitol, erythritol, sorbitol), and combinations thereof.

Proteins suitable for use in the compositions of the present disclosure, in addition to the *Salacia oblonga* extract component as described herein, include hydrolyzed, partially hydrolyzed or non-hydrolyzed proteins or protein sources, and can be derived from any known or otherwise suitable source such as milk (e.g., casein, whey), animal (e.g., meat, fish), cereal (e.g., rice, corn), vegetable (e.g., soy), or combinations thereof.

Fats suitable for use in the compositions of the present disclosure include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, and combinations thereof.

The concentration or amount of fat, protein, and carbohydrate in the compositions of the present disclosure may vary considerably depending upon the particular product form (e.g., solid, liquid, powder) and the various other formulations and targeted dietary needs. These macronutrients are most typically formulated within any of the caloric ranges (embodiments A-F) described in the following table (each numerical value is preceded by the term "about").

| **Macronutrient** | **Embodiment A** | **Embodiment B** | **Embodiment C** |
|---|---|---|---|
| **Carbohydrate** | 0-98 | 2-96 | 10-75 |
| % Total Calories | | | |
| **Protein** | 0-98 | 2-96 | 5-70 |
| % Total Calories | | | |
| **Fat** | 0-98 | 2-96 | 20-85 |
| % Total Calories | | | |

| | **Embodiment D** | **Embodiment E** | **Embodiment F** |
|---|---|---|---|
| **Carbohydrate** | 30-50 | 25-50 | 25-50 |
| % Total Calories | | | |
| **Protein** | 15-35 | 10-30 | 5-30 |
| % Total Calories | | | |
| **Fat** | 35-55 | 1-20 | 2-20 |
| % Total Calories | | | |

### Optional Ingredients

The nutritional compositions may further comprise other optional components that may modify the physical, chemical, aesthetic or processing characteristics of the products or serve as pharmaceutical or additional nutritional components when used in a targeted population. Many such optional ingredients are known or otherwise suitable for use in nutritional products and may also be used in the compositions herein, provided that such optional ingredients are safe and effective for administration and are compatible with the essential and other selected components in the compositions.

Non-limiting examples of such other optional ingredients include preservatives, antioxidants, buffers, pharmaceutical actives, sweeteners, colorants, flavors, flavor enhancers, thickening agents and stabilizers, emulsifying agents, lubricants, and so forth.

The nutritional compositions may further include one or more minerals, non-limiting examples of which include phosphorus, sodium, chloride, magnesium, manganese, iron, copper, zinc, iodine, calcium, potassium, chromium, chromium picolinate, molybdenum, selenium, and combinations thereof.

The nutritional compositions may also include one or more vitamins, non-limiting examples of which include carotenoids (e.g., beta-carotene, zeaxanthin, lutein, lycopene), biotin, choline, inositol, folic acid, pantothenic acid, choline, vitamin A, thiamine (vitamin B1), riboflavin (vitamin B2), niacin (vitamin B3), pyridoxine (vitamin B6), cyanocobalamin (vitamin B12), ascorbic acid (vitamin C), vitamin D, vitamin E, vitamin K, and various salts, esters or other derivatives thereof, and combinations thereof.

### Manufacture

The nutritional compositions of the present disclosure may be prepared by any known or otherwise effective manufacturing technique for preparing the selected product form. Many such techniques are known for any given product form such as nutritional liquids or nutritional bars and can easily be applied by one of ordinary skill in the art to the nutritional compositions described herein.

The nutritional compositions of the present disclosure can likewise be prepared by any known or otherwise effective manufacturing technique for preparing the various pharmaceutical product forms. Many such techniques are known for any given pharmaceutical product form such as capsules, tablets, liquids, and so forth, and can easily be applied by one of ordinary skill in the art to the compositions described herein.

As a basic liquid formulation, the nutritional compositions of the present disclosure may be prepared by dissolving the *Salacia oblonga* extract in water or a dilute acid solution to form a liquid embodiment of the present disclosure.

As a basic solid formulation, the nutritional compositions of the present disclosure may be prepared by combining a *Salacia oblonga* extract along with any tablet forming materials or other excipients, and then dry blending the powders prior to processing it into the desired solid product form, e.g., tablet, capsule, caplet, and so forth.

In yet another embodiment, the nutritional compositions of the present disclosure may be formulated as a nutritional liquid, including a juice or milk or soy-based liquid, comprising the *Salacia oblonga* extract. Such an embodiment may be prepared by first forming an oil and fiber blend containing all formulation oils, any emulsifier, fiber and fat-soluble vitamins. Additional slurries (typically a carbohydrate and two protein slurries) are prepared separately by mixing the carbohydrate and minerals together and the protein in water. The slurries are then mixed together with the oil blend. The resulting mixture is homogenized, heat processed, standardized with any water-soluble vitamins, flavored and the liquid terminally sterilized or aseptically filled or dried to produce a powder.

Other product forms such as nutritional bars may be manufactured, for example, using cold extrusion technology as is known and commonly described in the bar manufacturing art. To prepare such compositions, typically all of the powdered components are dry blended together, which typically includes the *Salacia oblonga* extract and any proteins, vitamin premixes, certain carbohydrates, and so forth. The fat-soluble components are then blended together and mixed with any powdered premixes. Finally any liquid components are then mixed into the composition, forming a plastic like composition or dough. The resulting plastic mass can then be shaped, without further physical or chemical changes occurring, by cold forming or extrusion, wherein the plastic mass is forced at relatively low pressure through a die, which confers the desired shape. The resultant exudate is then cut off at an appropriate position to give products of the desired weight. If desired the solid product is then coated, to enhance palatability, and packaged for distribution.

The solid nutritional embodiments of the present disclosure may also be manufactured through a baked application or heated extrusion to produce solid product forms such as cereals, cookies, crackers, and similar other product forms. One knowledgeable in the nutrition manufacturing arts is able to select one of the many known or otherwise available manufacturing processes to produce the desired final product.

The compositions of the present disclosure may, of course, be manufactured by other known or otherwise suitable techniques not specifically described herein without departing from the spirit and scope of the present disclosure. The present embodiments are, therefore, to be considered in all respects as illustrative and not restrictive and that all changes and equivalents also come within the description of the present disclosure. The following non-limiting examples will further illustrate the compositions and methods of the present disclosure.

### Methods of Use

The nutritional compositions as described herein may be used with individuals afflicted with or otherwise at risk of developing impaired glucose tolerance or diabetes, especially type 2 diabetes. These compositions can also be administered, however, in any individual as a nutrition source, especially in those in whom a blunted glycemic response is desirable. Methods of using the compositions including the *Salacia oblonga* extract include methods of administering the composition once daily, twice daily, three times a day, four times a day or even more times per day. Methods of using the compositions including the *Salacia oblonga* extract also include administering the composition before or after a meal.

The *Salacia oblonga* extract as described herein can be utilized in a number of methods in accordance with the present disclosure. In one embodiment, an amount of *Salacia oblonga* extract is included in a nutritional composition administered to a person with glucose intolerance, a prediabetic or type 2 diabetic individual to delay the onset or progression of type 2 diabetes in the subject.

In another embodiment, an amount of *Salacia oblonga* extract is included in a nutritional composition administered to a person with glucose intolerance, a prediabetic or type 2 diabetic individual to enhance and prolong intestinal incretin release, mainly GLP 1, levels to allow for enhanced β-cell insulin secretion when the blood glucose concentration is elevated above basal levels.

In another particular embodiment, an amount of *Salacia oblonga* extract is included in a nutritional composition administered to a person with glucose intolerance, a prediabetic or type 2 diabetic individual to modify feeding behavior and food intake, and thereby with the potential value in regulating appetite and stabilizing body weight in obese patients. *Salacia oblonga* mobilizes GLP-1 release from distal segment, which activates a neural pathway to cause sensation of fullness and limitation of food intake by delaying gastric emptying.

In another particular embodiment, an amount of *Salacia oblonga* extract is included in a nutritional composition administered to a person with glucose intolerance, a prediabetic, or type 2 diabetic individual to reduce glucagon secretion in the individual. Because glucagon is a hormone that causes the liver to convert stored glycogen into glucose and release the glucose into the bloodstream, by reducing the production/secretion of glucagon in the pancreas, the amount of glucose in the bloodstream is reduced.

The compositions and methods of the present disclosure may be directed to any individual, including humans and other mammals such as dogs, cats, rodents, cows, sheep, swine, goats, horses and other hoofed animals, and so forth. Healthy individuals at risk of type 2 diabetes may be administered the compositions as well. The methods of the present disclosure may be employed such that the composition including the *Salacia oblonga* extract is administered before, during, or after carbohydrate intake (such as from a meal, drink, or snack) to improve glucose tolerance and reduce the glycemic response. In one embodiment, the administration of the *Salacia oblonga* extract composition is conducted within about one hour of carbohydrate consumption by the subject. In another embodiment, the administration of the *Salacia oblonga* extract composition is conducted within about 30 minutes of carbohydrate consumption by the subject.

The methods of the present disclosure may be used to treat and/or control glucose tolerance, diabetes, obesity, and/or symptoms and side effects of glucose tolerance, diabetes, and obesity. Specifically, the compositions may be used to treat type 2 diabetes. Symptoms and side effects of diabetes include one or more of high blood glucose levels, sleep habits such as insomnia, general energy level such as lethargy, strength, body weight/poor or increased appetite, reflux, irregularity, stomach neuropathy, kidney failure, heart disease, stroke, and deteriorating eyesight.

### EXAMPLES

The following Examples illustrate specific embodiments and/or features of the compositions and methods of the present disclosure. The Examples are given solely for the purpose of illustration and are not to be construed as limitations, as many variations thereof are possible without departing from the spirit and scope of the disclosure.

### Example 1

In this Example, the acute and chronic effect of *Salacia oblonga* extract on the glycemic and insulinemic responses in a meal tolerance test are analyzed.

Acute effect was performed in Obese Zucker rats at the age of 15 wks. Twenty obese Zucker rats were weighted and divided into 2 treatment groups of ten animals. Controls group was orally treated with a solution containing maltodextrin (1g/kg body weight). *Salacia oblonga* groups was orally treated with the same maltodextrin solution but supplemented with the extract, at the dose of 150 mg/kg body weight. Blood samples were obtained via tail vein at baseline and at 15, 30, 60, 90, 120 and 180 min postprandial, for glucose analysis. Glucose was determined directly using Precision G equipment (Medisense, Bedford, Massachusetts, USA).

Chronic effect was performed in Obese Zucker rats at the age of 15 wks. Twenty obese Zucker rats were weighed and assigned among one of two treatments (n=10 per treatment). The control group received a standard purified rodent diet (AIN-93M) ad libitum for 8 weeks. *Salacia oblonga* (SOE) group received, during the same period, the standard diet supplemented with the *Salacia oblonga* extract (at the concentration of 1.13 g extract per kg diet). The individual body weights were recorded at the start of the study and at weekly intervals thereafter. The food consumption of each rat was measured daily. Considering the daily dietary intake, the average amounts of *Salacia* extract consumed per animal were about 25 mg Salacinol per day. At 7 wks of treatment, after an overnight fasting, control and *Salacia* groups underwent a meal tolerance test using Maltodextrin (1g/kg body weight). Blood samples were withdrawn from the tail vein at 0 (just before MD challenge) and at 15, 30, 60, 90, 120 and 180 min for the determination of postprandial glycemic and insulinemic responses. Glucose was determined directly using Precision G equipment (Medisense, Bedford, Massachusetts, USA). Serum insulin concentration was measured by enzyme immunoassay, using rat insulin as standard (ALPCO-Mercodia Ultra sensitive rat insulin ELISA).

### Statistical analysis

Results were expressed as means ± SEM. Two-way ANOVA, considering the terms treatment and time, was used to analyze the repeated measured design for glucose and insulin. A *posteriori* comparisons were done between treatments at each time point using the test of Tukey. Incremental area under the curve was calculated using the trapezoid method and differences between treatments were analyzed using Student's t- test.

### Results

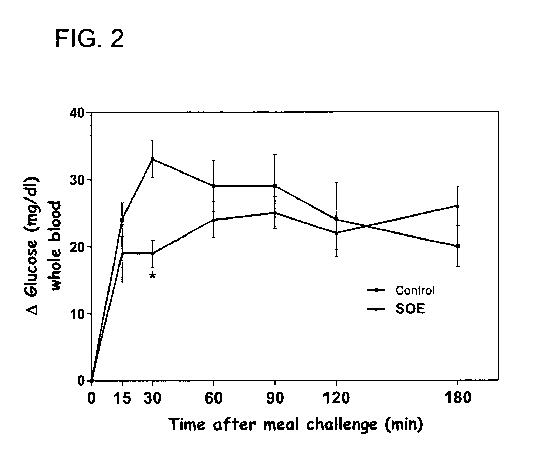

Acute effect.: Figure 2 displays the incremental blood glucose concentrations, in 15 wk old obese Zucker rats, after the meal oral challenge using maltodextrin supplemented with Salacia extract. In both groups the first increase in blood glucose concentration was detected at 15 min after the meal oral challenge. At 30 min post-gavage the glucose peak was, significantly lower in the group treated with *Salacia* extract compared to the group treated only with maltodextrin

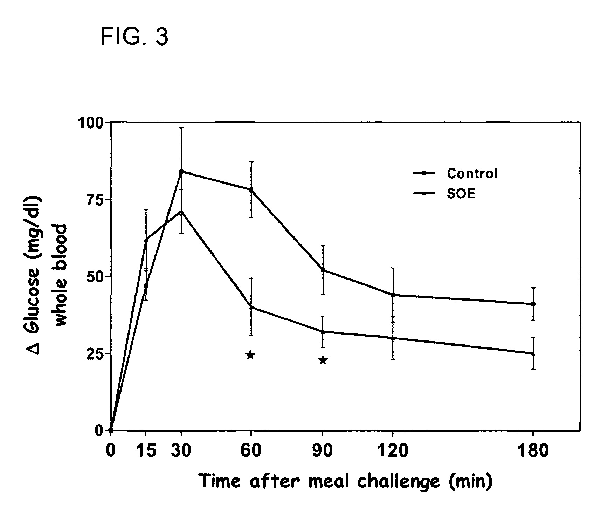

Chronic effect: After 7 weeks of treatment, the control and Salacia groups underwent a meal tolerance test using maltodextrin (1g/kg body weight) (Figure 3). In all the rats, the concentration of glucose in blood increased after 15 min of oral administration of maltodextrin solution. At 30 min, blood glucose level, in absolute terms, was higher in the control rats as compared to the group treated with *Salacia oblonga.* At 60 min, Salacia group showed lower glucose levels in comparison to the untreated rats, being this decrease statistically significant.

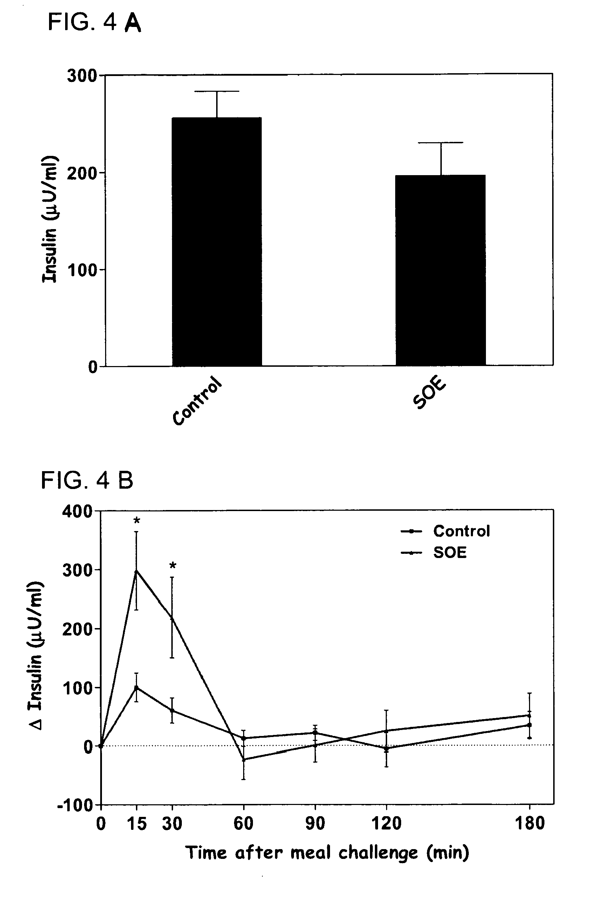

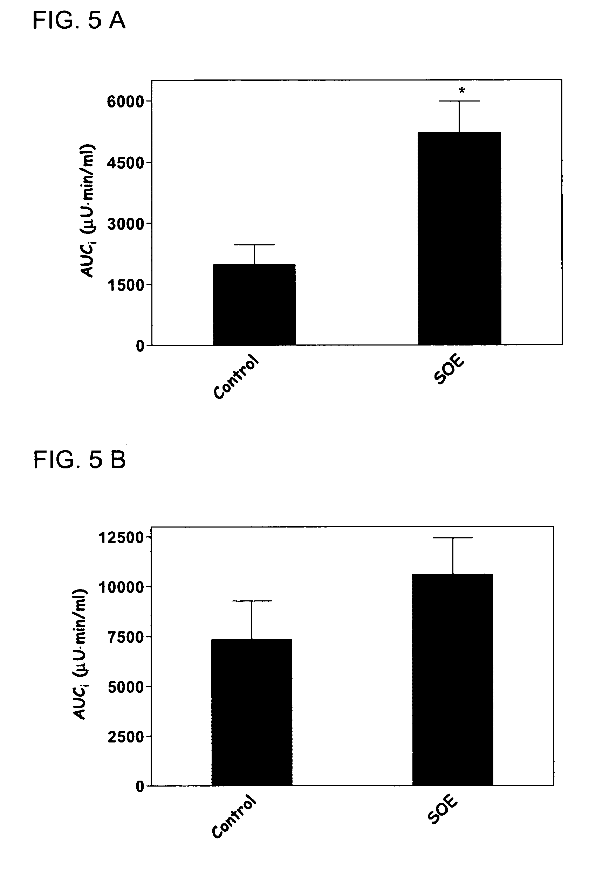

Figure 4 (A) displays the fasting insulin concentration of rats chronically treated with *Salacia oblonga* extract for 8 wks. *Salacia oblonga* extract induced a decrease in the insulin basal levels in comparison to the control rats, although these differences were not statistically significant. The insulinemic responses after an oral meal challenge using maltodextrin are showed in the Figure 4 (B). In all rats the concentration of insulin increased after 15 min of challenge. *Salacia oblonga* significantly increased the insulinemic response as compared to the control group at both 15 and 30 min. Furthermore, the AUC for insulin, between 0 and 30 min post-gavage, was significantly higher for the Salacia oblonga group than for control group (Figure 5A). However, this effect was not found when the AUC was calculated between 0 and 180 min post-gavage (Figure. 5B)

### Conclusion

Acute and chronic administration of Salacia extracts for 8 weeks improved the postprandial glycemic response after an oral meal challenge with maltodextrin, in obese Zucker rats. In this chronic study, the hypoglycemic activity of Salacia oblonga is mediated by enhancing insulin secretion.

### Example 2

In this Example, the effect of *Salacia oblonga* extract on tissue sensitivity to insulin in obese Zucker rats (OZR) is analyzed using the euglycermic-hyperinsulinemic clamp technique.

At the age of 15 wks, twenty male obese Zucker rats (fa/fa) (OZR) are assigned into two treatments groups (n=10). The control group receives the standard purified rodent diet, manufactured according to AIN-93M guidelines, *ad libitum* for 8 weeks. The *Salacia oblonga* (SOE) group receives, during the same period, the standard diet supplemented with *Salacia oblonga* extract (commercially available from Tanabe Seiyaku Company Limited Osaka Japan) (Lot 02305) at the concentration of 1.35g extract/kg diet. Individual body weights are recorded at the start of the study and at weekly intervals thereafter. Food consumption for each rat is measured daily. Considering the daily dietary intake, the average amount of *Salacia oblonga* extract consumed is about 45 mg/Kg body weight. The day the euglycemic clamp experiment is performed, rats are food-deprived for 18 hours and anesthetized with 50 mg Pentothal/kg body weight. Two catheters are placed in the right jugular vein: one for insulin infusion and one for glucose infusion. Another catheter is placed in the left carotid artery for blood sampling. A tracheotomy is performed to allow for tracheal clearing. Throughout the experimental period, the body temperature of the anesthetized rats is maintained at 36.5-37.5°C using an electric blanket. After about 40 minutes of surgery, arterial blood samples are collected for determination of glucose and insulin basal concentrations. At time-0 human insulin is infused (15 mU/kg.min). Blood samples are subsequently drawn at 5-minute intervals for determination of blood glucose (Precision G Medisense, Bedford, Massachusetts, USA). An infusion of 30% glucose is adjusted to maintain blood glucose at a level of about 100 mg/dl. Steady state is ascertained when a fixed glucose infusion rate (GIR) maintains blood glucose measurements constant for at least 30 minutes. Under this steady state, the GIR equals glucose uptake by all the tissues in the body and is therefore a measure of tissue sensitivity to exogenous insulin.

### Statistical analysis

Data are expressed as mean ± SEM. Two way ANOVA, considering the terms treatment and time, is used to analyze the data for body weight and GIR. A *posteriori* comparisons are done between treatments at each time point using the test of Bonferoni. Incremental area under the curve is calculated using the trapezoid method and differences between treatments are analyzed using Student's t-test. T-test is also used to evaluate the differences between treatment for fasting glucose and insulin levels.

### Results

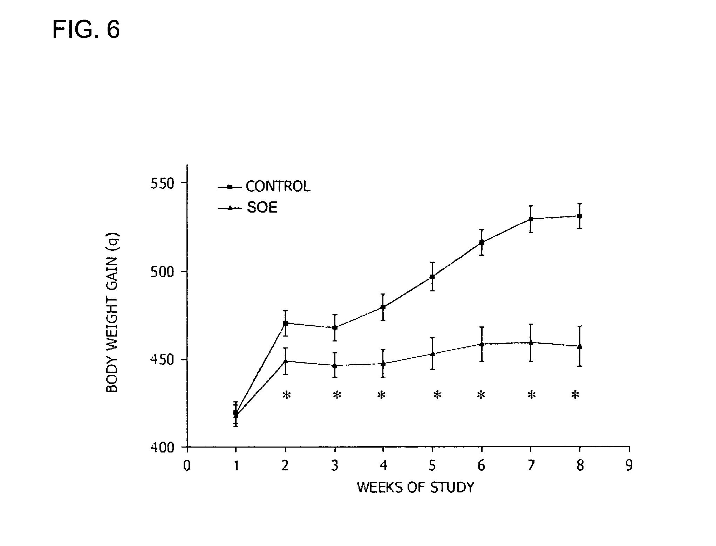

Initial body weight is similar between the two groups (See Figure 6). Rats consuming the diet supplemented with *Salacia oblonga* extract have reduced weight gain compared with rats consuming the control diet. At the end of the experiment, rats consuming the control diet gained more weight (112 ± 4 g) compared with rats consuming *Salacia oblonga* extract (39 ± 8 g). Food intake increases over time in the control group. On average, over the 8 week experiment, rats in the control group consume 23.1 ± 1.3 g/day compared to 15.3 ± 1 g/day for rats consuming *Salacia oblonga* extract.

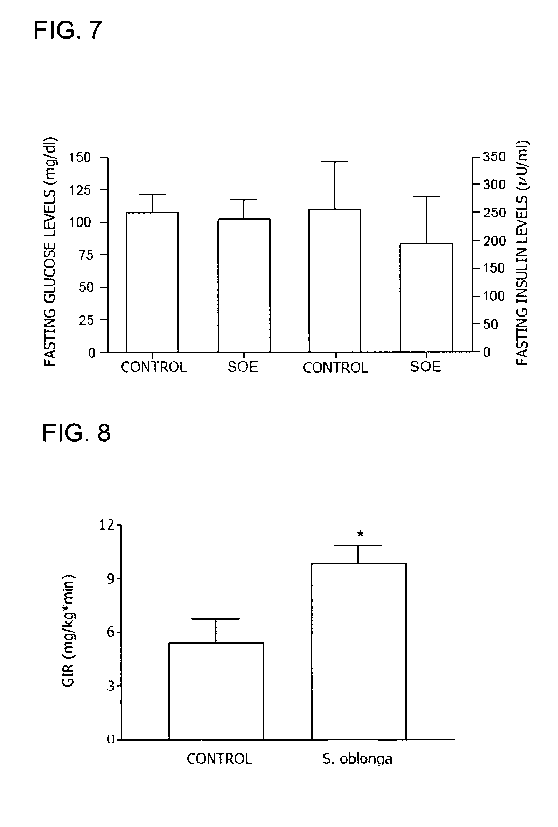

Figure 7 displays the fasting glucose and insulin concentrations of rats chronically treated with *Salacia oblonga* extracts for 8 wks. Basal glucose levels are not affected by *Salacia oblonga* extract treatment. However, *Salacia oblonga* extract induces a decrease in the insulin basal levels in comparison to the control rats, although these differences are not statistically significant. This mild insulin reduction could be a consequence of the improvement of insulin responsiveness by peripheral tissues mediated by *Salacia oblonga* extract.

To determine the whole-body insulin sensitizing effect of *Salacia oblonga* extract in obese Zucker rats with marked insulin resistance, an euglycemic clamp technique is performed (Figure 8). The insulin levels during the steady-stage period are not significantly different between the two study groups (849 ± 190 and 853 ± 180 µU/ml Control vs SOE groups), indicating that the hyperinsulinemic clamp is reached to examine glucose infusion rate (GIR). The quantity of glucose required to establish euglycemia during this period is affected by diet. The GIR required to maintain euglycemia is 45% greater in rats fed with the *Salacia oblonga* extract supplemented diet than in those fed with the control diet. This result shows that *Salacia oblonga* extract is able to stimulate glucose tissue disposal as an insulin-sensitizing agent.

### Example 3

In this Example, the effect of *Salacia oblonga* extract on β-cell sensitivity to glucose in obese Zucker rats (OZR) is analyzed using the hyperglycemic clamp technique.

At the age of 15 wks, twenty OZR are assigned into two treatments groups (n=10). The control group receives the standard purified rodent diet as described in Example *1 ad libitum* for 3 weeks. The *Salacia oblonga* extract group receives, during the same period, the standard diet supplemented with the *Salacia oblonga* extract at a concentration of 3 g extract/kg diet. Individual body weights are recorded at the start of the study and at weekly intervals thereafter. Food consumption for each rat is measured daily. Considering the daily dietary intake, the average amount of *Salacia oblonga* extract consumed is about 125 mg/kg body weight. The day of the hyperglycemic clamp experiment, rats are food deprived for 18 hours and anesthetized with 50 mg Pentothal/kg body weight. One catheter is introduced into the right jugular vein for glucose infusion. Another catheter is placed in the left carotid artery for blood sampling. A tracheotomy is performed to allow for tracheal clearing. Throughout the experimental period, the body temperature of the anesthetized rats is maintained at 36.5-37.5°C using an electric blanket. After about 40 minutes of surgery, arterial blood samples are collected for determination of glucose and insulin basal concentrations. At 0-time, blood glucose concentration is acutely raised to 250 mg/dl by a priming infusion of glucose (30% glucose). This hyperglycemic plateau is subsequently maintained by adjustment of a variable glucose infusion, for 120 minutes. Steady state is ascertained when a fixed glucose infusion rate (GIR) maintains the hyperglycemic plateau for at least 30 minutes. Blood samples are subsequently drawn at 5-minutes intervals for the determination of blood glucose and serum insulin concentrations (Precision G Medisense, Bedford, Massachusetts, USA). Under these conditions of constant hyperglycemia the serum insulin response is biphasic with an early burst of insulin release during the first 10 minutes (early phase) followed by a gradually progressive increase in insulin concentration (10-120 minutes, early-late phase). The hyperglycemic clamp technique allows for the assessment of two important physiological variables. First, it allow for the quantification of the β-cells response as well as the independent evaluation of the early and late phases of insulin secretion, and second the determination of the amount of glucose metabolized by the body (GIR in the steady-state).

### Statistical analysis

Data are expressed as mean ± SEM. Similar to Example 1, two way ANOVA, considering the terms treatment and time, is used to analyze the data for body weight and GIR. *A posteriori* comparisons are done between treatments at each time point using the test of Bonferoni. Incremental area under the curve is calculated using the trapezoid method and differences between treatments are analyzed using Student's t-test. T-test is also used to evaluate the differences between treatment for fasting glucose and insulin levels.

### Results

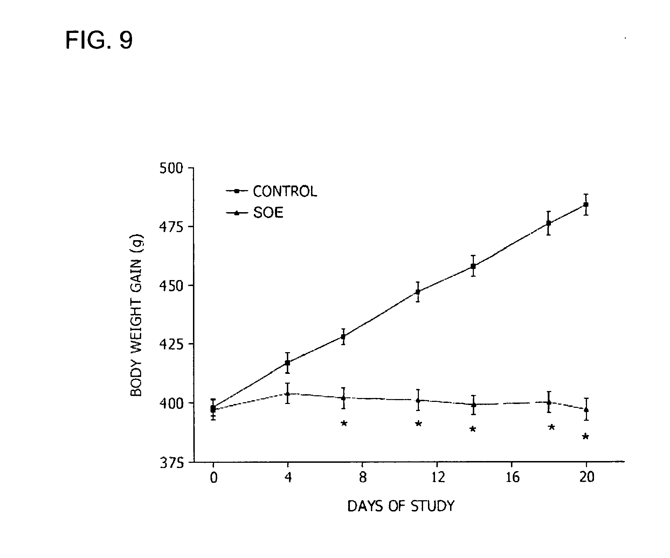

Initial body weight is similar between the two groups (See Figure 9). Body weight gain is totally suppressed in rats that consumed the diet supplemented with *Salacia oblonga* extract for 21 days in comparison to the control group (body weight gain 89 ± 14 g). Daily food intake is higher in the control group (25 ± 1.0 g/day) as compared to the *Salacia oblonga* extract group (14.8 ± 0.7 g/day).

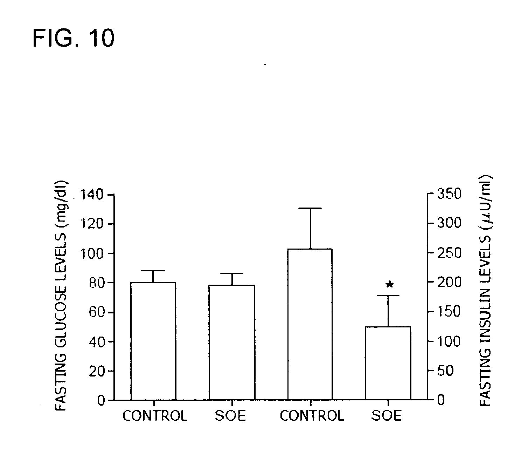

Fasting glucose and insulin concentrations of rats chronically treated with *Salacia oblonga* extracts for 21 days are shown in Figure 10. Basal glucose levels do not differ between dietary groups. However, fasting insulin concentration is significantly lower in rats fed with *Salacia oblonga* extract as compared to control rats. This insulin reduction could be due to the improvement of insulin responsiveness by peripheral tissues, mediated by *Salacia oblonga* extract as it is demonstrated by the euglycemic clamp study of Example 2.

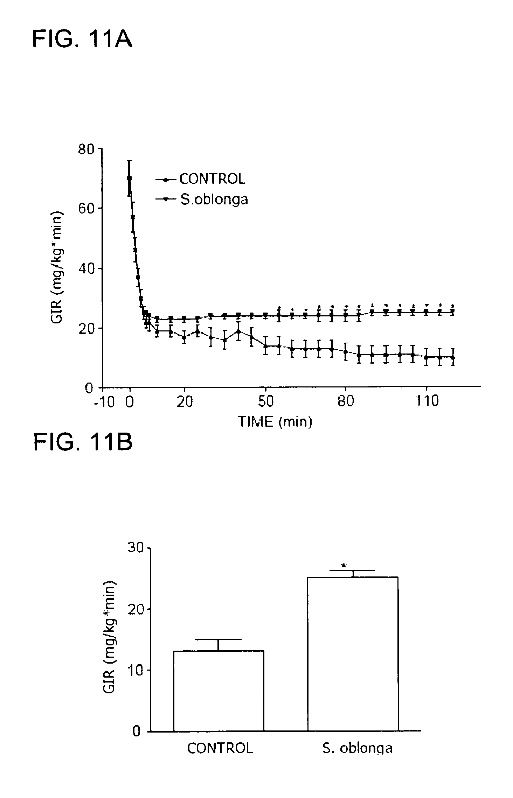

To assess the potential ability of *Salacia oblonga* extract to modulate glucose tolerance as well as β-cell sensitivity to glucose, a hyperglycemic clamp technique is conducted in obese Zucker rats. In the steady-state period, the GIR is higher in the rats chronically treated with *Salacia oblonga* extract than in the control rats (See Figures 11A and 11B). This result shows that *Salacia oblonga* extract is able to improve glucose tolerance in animals with severe insulin resistance.

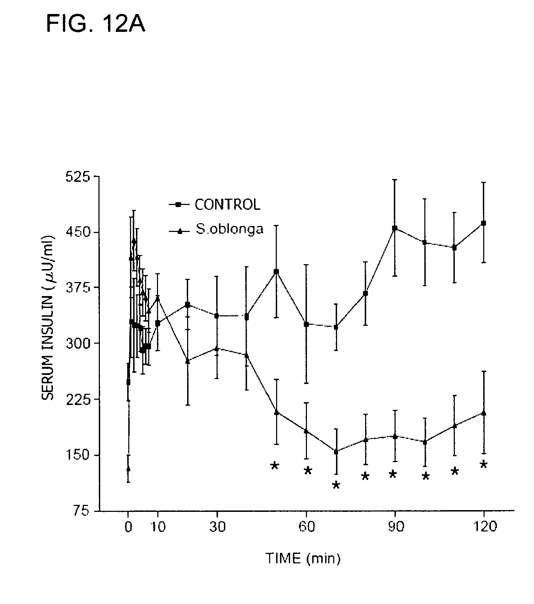

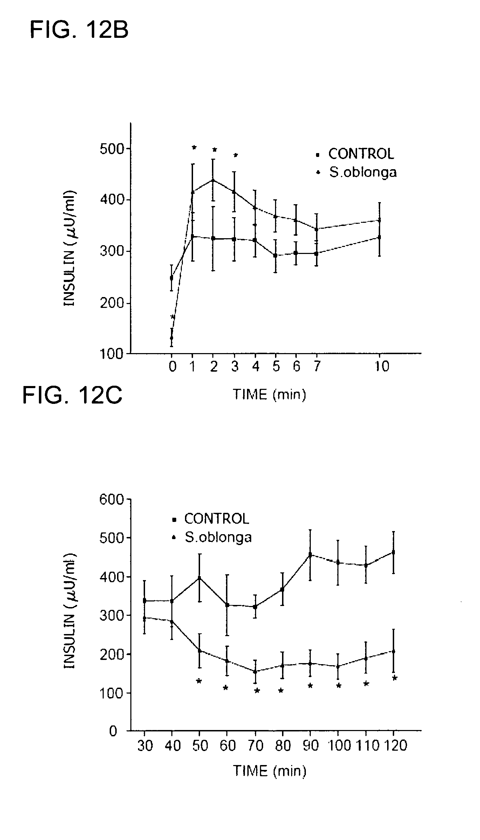

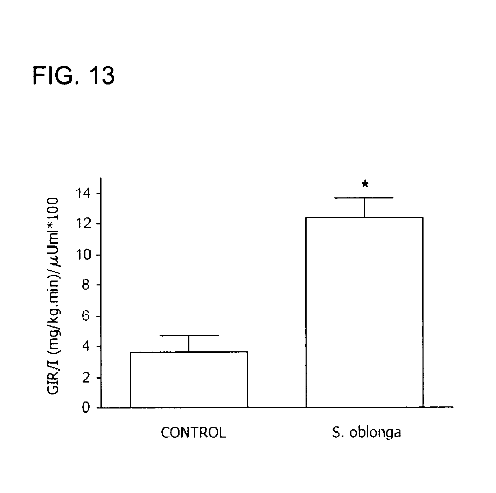

Serum insulin response under hyperglycemic conditions, as a measure of the β-cell response to glucose, is shown in Figures 12A-12C. In control rats, the insulin response to the sustained hyperglycemia is biphasic, with an early phase of insulin release (0-10 minutes) followed by a late phase of insulin secretion until the end of the study (30-120 minutes). Chronic treatment *of Salacia oblonga* extract induces an increase of the initial phase of insulin release and a decrease in the insulin concentrations that lasts until the end of the study, as compared to the control group. Moreover, under the hyperglycemic clamp conditions, the ratio of GIR/Insulin during 20 to 120 minutes (Figure 13) is a measure of the quantity of glucose metabolized per unit of plasma insulin concentration, and is thus a reasonable index of tissue sensitivity to endogenously secreted insulin. GIR/Insulin ratio is four times greater in the rats fed with *Salacia oblonga* extract in comparison to control rats (See Figure 9), indicating that *Salacia oblonga* extract increases peripheral glucose utilization.

### Conclusion

The combined results of Examples 2 and 3 show that *Salacia oblonga* extract induces a dose-dependent reduction of insulin concentration in fasting states, indicating an improvement in peripheral insulin action. The insulin-sensitizing effect of the extract is further supported by the results of Example 2.

Furthermore, the results of Example 2 demonstrate that chronic administration of *Salacia oblonga* extract significantly improves glucose tolerance in a model of insulin resistance. This anti-hyperglycemic action is exerted not only at the luminal level by inhibiting α-glucosidase activities but also at the systemic level by increasing tissue insulin sensitivity and stimulating β-cells response to glucose. *Salacia oblonga* extract may therefore be a convenient and effective long-term option for the treatment of insulin resistance and type 2 diabetes, with the added benefits of reducing weight loss.

### Example 4

In this Example, the insulin secretagogue effect of *Salacia oblonga* extract is verified in pancreatic-isolated islets using static incubation methodology. Furthermore, the potential capacity of *Salacia oblonga* extract to modulate β-cell sensitivity to glucose is examined in islets isolated from, obese insulin resistance rats.

Pancreatic islets are isolated from the pancreas of a specimen rat by collagenase digestion. Specifically, non-fasting rats are anaesthetized and bled through cardiac puncture. The pancreas is visible through an incision made in the ventral abdomen, and the common bile duct is exposed. The point at which the common bile duct enters the duodenum is clamped off, and a small hole is made in the connective tissue duct. The pancreas is distended with approximately 10.0 mL Hank's Balanced Salt Solution (HBSS) through a cannule, and is removed in one piece. The tissue taken from two rats is minced with a scissors and incubated with collagenase P (44.4 U/pancreas), and saturated with O₂/CO₂ (95/5, v/v) for 10 minutes at 37°C in 50 ml siliconized borosilicate tube. The tube is shaken by hand for 2 minutes at 37°C and then maintained for 6 minutes at room temperature. Enzymatic digestion is stopped with 30 mL HBSS- 10% FBS. The pancreatic suspension is diluted with HBSS (100 ml), and the mixture of non-digested/digested tissue is separated by filtration (pore size; 500 µm). Digested suspension is washed with HBSS and brought to a final volume close to 150 ml, and placed in a glass container. When the islets have sedimented at the bottom of the container, the supernatant is removed and replaced by an equal volume of HBSS solution. This procedure is repeated two or three times in order to remove the bulk of the exocrine tissue, which sediments more slowly than the islets. The final washed preparation is poured into a Petri dish for collection of the islets. The islets are collected by handpicking under a dissecting microscope, providing 40-fold of magnification. The islets appear as free, round or ovoid structures with greyish white to brownish red color.

Groups of 7 freshly collected islets are incubated for 90 minutes at 37°C under shaking (150 min-1) in 1.0 mL of Krebs-Ringer bicarbonate buffer (KRB, equilibrated with 5%CO₂/95%O₂), supplemented with 0.5% bovine serum albumin, glucose 16.7 mM, and *Salacia oblonga* extract (available from Tanabe Seiyaku Company Limited (Osaka Japan) as Lot 020305). *Salacia oblonga* extract is tested in the presence of 16.7 mM glucose at two concentrations, 0.5 and 1 mg/ml. Immediately after incubation, three buffer aliquots of each sample are collected and stored at -80°C for the later determination of insulin. Insulin released into the medium is measured by ELISA assay.

### Statistical analysis

All results are expressed as mean ± SD. Statistical significance is evaluated by one-way ANOVA, followed by Bonferroni comparisons test. The level of significance is set at p<0.05.

### Results

### Effect of Salacia oblonga on insulin release in insulin resistance obese Zucker rats

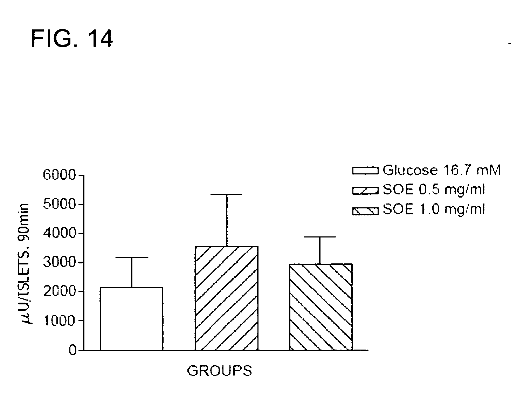

Insulin release capacity of intact islets isolated from obese Zucker fa/fa rats (OZR), a model of insulin resistance genetically induced, was not significantly affected by the addition of *Salacia oblonga* extract to the culture medium. (Figure 14)

### Conclusion

Insulin secretagogue activity of *Salacia oblonga* extract determined using the hyperglycaemic clamp technique, was not statistically reproduced in pancreatic β-cells isolated from chronically treated OZR. These contradictory in vivo, hyperglycemic clamp, and in vitro , static incubation, results on insulin secretion may be only explained considering that Salacia oblonga may exert an incretin effect enhancing and prolonging of GLP 1 release in the intestinal distal segment.

As can be seen from Examples the *in vivo* experiments show that chronic *Salacia oblonga* extract treatment stimulated the initial phase of insulin release during the first ten minutes of the hyperglycemic clamp. These data show that *Salacia oblonga* extract has an insulin secretagogue effect by stimulation of the early phase of insulin secretion. The *in vitro* study, Example 4, utilizing pancreatic-isolated islets, however, showed that *Salacia oblonga* extract treatment failed to demonstrate an effect on insulin release. These results show that *Salacia oblonga* extract exerts an incretin effect.

## Claims

1. A method of delaying the progression of type 2 diabetes in a subject, the method comprising administering to the subject a nutritional composition comprising an effective amount of a *Salacia oblonga* extract.

2. The method as set forth in claim 1 wherein the composition is an aqueous emulsion.

3. The method as set forth in claim 1 wherein the composition is reconstituted from a powdered composition.

4. The method as set forth in claim 1 wherein the composition further comprises a fat, a protein, and a carbohydrate.

5. The method as set forth in claim 1 wherein the composition comprises from about 0.001% to about 1.0% (by weight nutritional composition) *Salacia oblonga* extract.

6. The method as set forth in claim 1 wherein the composition comprises from about 0.001% to about 0.25% (by weight nutritional composition) *Salacia oblonga* extract.

7. The method as set forth in claim 1 wherein the composition is administered such that the daily intake of *Salacia oblonga* extract is at least about 150 mg/kg body weight per day.

8. The method as set forth in claim 1 wherein the composition is administered such that the daily intake of *Salacia oblonga* extract is at least about 175 mg/kg body weight per day.

9. The method as set forth in claim 1 wherein the nutritional composition is chronically administered to the subject for a period of at least about 4 weeks.

10. The method as set forth in claim 1 wherein the nutritional composition is chronically administered to the subject for a period of at least about 2 months.

11. A method of increasing the incretin effect in an individual afflicted with impaired glucose tolerance or diabetes, the method comprising administering to the subject a nutritional composition comprising an effective amount of a *Salacia oblonga* extract.

12. The method as set forth in claim 11 wherein the composition is an aqueous emulsion.

13. The method as set forth in claim 11 wherein the composition is reconstituted from a powdered composition.

14. The method as set forth in claim 11 wherein the composition further comprises a fat, a protein, and a carbohydrate.

15. The method as set forth in claim 11 wherein the composition comprises from about 0.001% to about 1.0% (by weight nutritional composition) *Salacia oblonga* extract.

16. The method as set forth in claim 11 wherein the composition comprises from about 0.001% to about 0.25% (by weight nutritional composition) *Salacia oblonga* extract.

17. The method as set forth in claim 11 wherein the composition is administered such that the daily intake of *Salacia oblonga* extract is at least about 150 mg/kg body weight per day.

18. The method as set forth in claim 11 wherein the composition is administered such that the daily intake of *Salacia oblonga* extract is at least about 175 mg/kg body weight per day.

19. A method of increasing glucagon-like peptide-1 activity in an individual inflicted with impaired glucose tolerance or diabetes, the method comprising administering to the subject a nutritional composition comprising an effective amount of a *Salacia oblonga* extract.

20. A method of decreasing glucagon secretion in a subject, the method comprising administering to the subject a nutritional composition comprising an effective amount of a *Salacia oblonga* extract.

21. A method of regulating satiety in a subject, the method comprising administering to the subject a nutritional composition comprising an effective amount of a *Salacia oblonga* extract.

## Amended claims

### Amended claims in accordance with Rule 137(2) EPC.

**1.** Use of an effective amount of a *Salacia oblonga* extract for the manufacture of a nutritional composition for delaying the progression of type 2 diabetes in a subject in need thereof.

**2.** The use as set forth in claim 1 wherein the composition is an aqueous emulsion.

**3.** The use as set forth in claim 1 wherein the composition is reconstituted from a powdered composition,

**4.** The use as set forth in claim 1 wherein the composition further comprises a fat, a protein, and a carbohydrate.

**5.** The use as set forth in claim 1 wherein the composition comprises from about 0.001% to about 1.0% (by weight nutritional composition) *Salacia oblonga* extract.

**6.** The use as set forth in claim 1 wherein the composition comprises from about 0.001% to about 0.25% (by weight nutritional composition) *Salacia oblonga* extract.

**7.** The use as set forth in claim 1 wherein the composition is administered such that the daily intake of *Salacia oblonga* extract is at least about 150 mg/kg body weight per day.

**8.** The use as set forth in claim 1 wherein the composition is administered such that the daily intake of *Salacia oblonga* extract is at least about 175 mg/kg body weight per day.

**9.** The use as set forth in claim 1 wherein the nutritional composition is chronically administered to the subject for a period of at least about 4 weeks.

**10.** The use as set forth in claim 1 wherein the nutritional composition is chronically administered to the subject for a period of at least about 2 months.

**11.** Use of an effective amount of a *Salacia oblonga* extract for the manufacture of a nutritional composition for increasing the incretin effect in an individual afflicted with impaired glucose tolerance or diabetes.

**12.** The use as set forth in claim 11 wherein the composition is an aqueous emulsion.

**13.** The use as set forth in claim 11 wherein the composition is reconstituted from a powdered composition,

**14.** The use as set forth in claim 11 wherein the composition further comprises a fat, a protein, and a carbohydrate.

**15.** The use as set forth in claim 11 wherein the composition comprises from about 0.001% to about 1.0% (by weight nutritional composition) *Salacia oblonga* extract.

**16.** The use as set forth in claim 11 wherein the composition comprises from about 0.001% to about 0.25% (by weight nutritional composition) *Salacia oblonga* extract.

**17.** The use as set forth in claim 11 wherein the composition is administered such that the daily intake of *Salacia oblonga* extract is at least about 150 mg/kg body weight per day.

**18.** The use as set forth in claim 11 wherein the composition is administered such that the daily intake of *Salacia oblonga* extract is at least about 175 mg/kg body weight per day.

**19.** Use of an effective amount of a *Salacia oblonga* extract for the manufacture of a nutritional composition for increasing glucagon-like peptide-1 activity in an individual inflicted with impaired glucose tolerance or diabetes.

**20.** Use of an effective amount of a *Salacia oblonga* extract for the manufacture of a nutritional composition for decreasing glucagon secretion in a subject.

**21.** Use of an effective amount of a *Salacia oblonga* extract for the manufacture of a nutritional composition for regulating satiety in a subject.
